# EUROPEAN PATENT SPECIFICATION

(11) **EP 4 247 416 B1**
(45) Date of publication and mention of the grant of the patent: **07.10.2026**
(21) Application number: 21827458.7
(22) Date of filing: 18.11.2021
(51) Int. Cl.: A61K 38/57, A61P 37/06, A61K 39/395, C07K 16/00

(54) **METHOD FOR TREATING ANTIBODY-MEDIATED REJECTION**
VERFAHREN ZUR BEHANDLUNG VON ANTIKÖRPERVERMITTELTER ABSTOSSUNG
MÉTHODES DE TRAITEMENT D'UN REJET À MÉDIATION PAR ANTICORPS

(30) Priority: 20.11.2020 US 202063116405 P
(43) Date of publication of application: 27.09.2023
(73) Proprietor: CSL Behring GmbH, 35041 Marburg (DE)
(72) Inventor: ROBERTS, John, Exton, Pennsylvania 19341 (US); UKNIS, Marc, Doylestown, Pennsylvania 18902 (US); VOIGT, Christine, Devon, Pennsylvania 19333 (US)
(74) Representative: CSL Global IP
(86) International application number: PCT/US2021/059872
(87) International publication number: WO 2022/109124

(56) References cited:
- US-A1- 2019 175 683
- US-B2- 9 895 428
- R. A. MONTGOMERY ET AL: "Plasma-Derived C1 Esterase Inhibitor for Acute Antibody-Mediated Rejection Following Kidney Transplantation: Results of a Randomized Double-Blind Placebo-Controlled Pilot Study", AMERICAN JOURNAL OF TRANSPLANTATION, vol. 16, no. 12, 27 June 2016 (2016-06-27), DK, pages 3468 - 3478, XP055583366, ISSN: 1600-6135, DOI: 10.1111/ajt.13871
- MEL BERGER ET AL: "Potential Roles for C1 Inhibitor in Transplantation :", TRANSPLANTATION, vol. 100, no. 7, 1 July 2016 (2016-07-01), GB, pages 1415 - 1424, XP055583370, ISSN: 0041-1337, DOI: 10.1097/TP.0000000000000995

## Description

### FIELD OF THE INVENTION

This disclosure relates to C1-INH for use in a method for treating antibody-mediated rejection (AMR) in a subject by administering C1 inhibitor (C1-INH) intravenously followed by subcutaneous administration according to a herein described dosing regimen. This disclosure further relates to C1-INH for use in a method for treating antibody-mediated rejection (AMR) in a subject by administering C1 inhibitor (C1-INH) subcutaneously only.

### BACKGROUND AND INTRODUCTION

Solid organ transplantation is one of the most challenging fields of modern medicine. It can be lifesaving for patients with end-stage kidney, heart, liver, lung or pancreas disease, but despite modern immunosuppression allograft loss due to organ rejection remains a major post-transplantation complication. Antibody-mediated rejection (AMR) has been recognized as one of the most important causes for graft loss (Davis et al., Transplant. Rev. 2017; 31(1): 47-54). Treatment of AMR with conventional therapies such as intravenous immunoglobulin (IVlg) or plasmapheresis is not always successful (Levine et al., Semin. Immunol. 2012; 24(2): 136-142). If patients do not respond to treatment, graft function generally declines progressively until the allograft is lost. Antibody-mediated rejection is mediated by donor-specific antibodies (DSA) directed against the allograft. These donor-specific antibodies can exist prior to transplantation or be produced *de novo* after transplantation. They often target human leukocyte antigen (HLA) molecules present on the vascular endothelium and tubules of the transplanted organ (Valenzuela et al., J. Clin. Invest. 2017; 127(7): 2492-2504; Cross et al., Front. Immunol. 2018; 9(106): 1-7; Irure et al., Transplant. Proc. 2016; 48(9): 2888-2890). Interaction between donor-specific antibodies and antigens of the allograft activates the classical pathway of the complement cascade. This activation significantly contributes to inflammation-mediated tissue damage of the allograft. Therefore, one approach that is being explored for treating of antibody mediated rejection is downregulation of the complement system (Levine et al., Semin. Immunol. 2012; 24(2): 136-142).

A glycoprotein that inhibits the classical pathway of the complement cascade is C1 inhibitor (C1-INH). It belongs to the protein family of serine protease inhibitors (serpins), which regulate the activity of serine proteases by inhibiting their catalytic activity (Bock et al., Biochemistry. 1986; 25(15): 4292-4301). Several studies have been disclosed that explore its use for treating antibody-mediated rejection. For example, in one study 20 IU/kg C1 inhibitor (Berinert^{®}) was administered intravenously to 10 patients intraoperatively and then twice weekly for 7 doses (NCT01134510; Vo et al., Transplantation. 2015; 99(2): 299-308). In a later study 20 IU/kg C1 inhibitor (Berinert^{®}) was administered to 6 patients intravenously on days 1, 2, and 3 and then twice weekly for 6 months (Viglietti et al., Am. J. Transplant. 2016; 16(5): 1596-1603). In a further study 9 subjects received C1 inhibitor (Cinryze^{®}) intravenously with an initial infusion of 5,000 IU on day 1, followed by 2,500 IU on days 3, 5, 7, 9, 11, and 13 (NCT01147302; Montgomery, Am. J. Transplant. 2016; 16(12): 3468-3478; cf. also EP 3 071 219 B1). This dosing regimen was also studied in a phase III clinical trial, but the trial was terminated prematurely when according to an interim analysis performed by the data monitoring committee it met the pre-specified criteria for futility (NCT02547220). A further study for intravenous administration of 100 IU/kg recombinant C1 inhibitor daily for 7 consecutive days was withdrawn prior to enrolling any patients (NCT01035593). The above studies show that although various dosing regimens for C1 inhibitor have been explored, there remains a need for improved methods of treating antibody-mediated rejection that may be used in cases where conventional therapy does not succeed.

### SUMMARY OF THE INVENTION

The inventors found an improved dosing regimen for treating antibody-mediated rejection. The invention is set out in the appended set of claims.

Further embodiments are provided in the disclosure as a whole.

### DESCRIPTION OF FIGURES

- Figure 1: Schematic view of the study designs of EXAMPLE 1. Five iv-doses of C1-INH (60 IU/kg) were administered intravenously to each subject over the first 13 days. Thereafter, C1-INH (60 IU/kg about twice weekly) was administered subcutaneously for 10 weeks to each subject. Treatment Period 1 of EXAMPLE 2 has an analogous design.
- Figure 2: Design of a clinical trial in accordance with EXAMPLE 2. The clinical trial comprises an open labeled Treatment Period 1 and a double-blind Treatment Period 2.
- Figure 3: Shows the mean eGFR at baseline (left) and at end of Treatment Period 1 (average of week 11 and 12 values, middle), along with mean change in eGFR from baseline to end of Treatment Period 1 as described in EXAMPLE1. Bars indicate standard error of the mean (SEM).

### DETAILED DESCRIPTION

The headings used herein are for organizational purposes only and are not to be construed as limiting the subject matter described.

### DEFINITIONS

Unless otherwise defined, scientific and technical terms used in connection with the present invention shall have the meanings that are commonly understood by those of ordinary skill in the art.

Unless otherwise indicated the International System of Units (SI) is used for units. Furthermore, any concentration range, percentage range, ratio range or integer range is to be understood to include the value of any integer within the recited range and, when appropriate, fractions thereof (such as one tenth and one hundredth of an integer).

In this application, the use of "or" means "and/or" unless stated otherwise. Further, unless otherwise required by context, singular terms shall include pluralities and plural terms shall include the singular.

As utilized in accordance with the present disclosure, the following terms, unless otherwise indicated, shall be understood to have the following meanings:
"About once weekly", "about twice weekly", and "about three times weekly" refer to a respective frequency of administration, wherein the term "about" indicates that it relates to an average frequency that has been rounded to integers (integer "1" for once weekly, integer "2" for twice weekly, etc.). For example, if 2 doses are administered per week for 2 weeks, then this corresponds to exactly twice weekly, which is encompassed by "about twice weekly over two weeks". If instead 2 doses are administered in the first week and 1 dose is administered in the second week, then this corresponds to an average of 1.5 doses per week, which would also be encompassed by the phrase "about twice weekly over two weeks" (since 1.5 is rounded to the next higher integer 2). A dosing range of "about twice to about three times weekly" over a period of several weeks encompasses dosing at an average number of doses per week that rounds to between 2 and 3 doses per week (e.g. from 1.5 to 3.4 doses per week).

"Administration" refers to the physical introduction of a formulation comprising an drugs into a subject using any of the various methods and delivery systems known to those skilled in the art. General routes of administration for drugs include intravenous, intramuscular, subcutaneous, intraperitoneal, spinal or other parenteral routes of administration, for example by injection. Administration can be performed, for example, once, a plurality of times, and/or over one or more extended periods. An "intravenous" administration means administration into a vein of a subject, in particular by injection. A "subcutaneous" administration means administration into subcutaneous tissue of a subject, in particular by injection. The term "injection" also encompasses injections over a prolonged time (infusions). Sites for subcutaneous administration may include the upper arm, the abdomen, the thigh, the upper back and the buttock.

"Antibody-mediated rejection" or "AMR" refers to the rejection of a transplant to a recipient due to the action of donor-specific antibodies (DSA) against the transplanted cells or tissue. Donor-specific antibodies may for example be directed against donor-specific HLA molecules, blood group antigen (ABO)-isoagglutinins, and/or endothelial cell antigens of an allograft. AMR may be categorized in accordance with the Banff classification as "active AMR" or "chronic active AMR". Criteria for diagnosing active AMR and chronic active AMR are defined in the revised Banff 2017 classification of AMR (Haas et al., Am. J. Transplant. 2018; 18(2): 293-307; Roufosse et al., Transplantation. 2018; 102(11): 1795-1814). As used herein, the terms "acute AMR", "acute/active AMR" and "active AMR" are interchangeable. The terms "chronic AMR" and "chronic active AMR" are also used interchangeably.

The term "refractory AMR" or "rAMR" as used herein refers to AMR that does not show improvement in organ function in response to therapy with immunomodulatory/immunosuppressive agents without complement inhibitors such as C1-INH. Generally, AMR is considered refractory when conventional therapy is not successful. For example, conventional therapy may be standard of care treatment (SOC) without C1-INH. For example, to determine the lack of improvement of organ function for kidneys, renal function may be evaluated by the methods known to a person skilled in the art, e.g. by taking eGFR into consideration. In some embodiments refractory AMR (rAMR) refers to AMR that does not show improvement in organ function in response to therapy that comprises administration of IVIg optionally with plasmapheresis (without administration of C1-INH).

According to the present invention, the terms "C1 inhibitor" or "C1-INH" refer to the proteins or fragments thereof that function as serine protease inhibitors and inhibit proteases associated with the complement system, for example proteases C1r and C1s as well as MASP-1 and MASP-2, with the kallikrein-kinin system, for example plasma kallikrein and factor XIIa, and with the coagulation system, for example factor Xla and factor XIIa. For further disclosure regarding the structure and function of C1-INH, see US 4,915,945, US 5,939,389, US 6,248,365, US 7,053,176, and WO 2007/073186.
"human C1-INH" or "hC1-INH" refer to a protein that comprises an amino acid sequence that is identical or essentially identical to the amino acid sequence of C1 inhibitor as naturally occurring in humans (circulating in human blood). Human C1-INH may be C1 inhibitor isolated from humans, for example gained from human plasma (human plasma-derived C1-INH also referred to as "pdC1-INH"). However, the term human C1-INH may also refer to a recombinant C1 inhibitor that comprises the same or essentially the same amino acid sequence as for C1 inhibitor naturally occurring in humans, but is not isolated from humans and instead produced recombinantly, e.g. by transfecting recombinant DNA into a host cell. In such a recombinant C1 inhibitor the amino acid sequence of naturally occurring human C1 inhibitor may also be fused to a half-life extending moiety. Some formulations of human C1-INH are commercially available under the trade names Berinert^{®} (plasma-derived), Cinryze^{®} (plasma-derived) or Ruconest^{®} (recombinant). In some embodiments the human C1-INH is a protein that consists of an amino acid sequence that is identical to the amino acid sequence of C1 inhibitor as naturally occurring in humans.

A "day" as understood herein relates to a time span of 24 hours, in particular reckoned from one midnight to the next, wherein the time zone of the location where the treatment is conducted determines midnight. A week, month, or year is divided into such days. Preferably, the terms day and "calendar day" may be used interchangeably. If a drug is administered over 2 days this does not necessarily mean that 24 hours between administrations have elapsed, but that the first dose is administered on (calendar) day 1 and the last dose on (calendar) day 2. However, a person skilled in the art often will administer drugs at about the same time of day, e.g. every morning between 8:00 and 10:00 am during patient visit. A "week" equals 7 days and a "month" is defined herein as 28 days (4 weeks).

A "dose" of C1-INH refers to a certain amount of C1-INH taken at one time, e.g. as intravenous or subcutaneous injection administered on a certain day. In some cases administration of a dose may take a significant amount of time, such as over several hours. In other cases an injection takes less time, e.g. within a few minutes to an hour. Any reference to "IU/kg", "mg/kg" or "g/kg" refers to the amount of drug (in U, mg, or g) in relation to the body weight of the subject (in kg) that receives the drug. It should be noted that if amounts for doses are provided, actual administered amounts may be rounded to the nearest practical volume and for reconstituted solutions this usually may correspond to administering whole milliliters of solution (not fractions), e.g. instead of 9.5 mL or 10.2 mL the nearest practical volume would be in both cases 10 mL. Furthermore, actual volume administered may instead also be rounded to the nearest lower practical volume, which relates to a volume which is rounded down from the actual volume (to avoid any higher dose than the calculated dose).

An "iv-dose" of C1-INH refers to a dose that is administered intravenously, i.e. the term not only refers to the dose as such, but also defines its mode of administration. Analogously, a "sc-dose" of C1-INH is a dose that is administered subcutaneously. Accordingly, the term "iv-dose" may be used interchangeably with "dose administered intravenously" and "sc-dose" may be used interchangeably with "dose administered subcutaneously". A different prefix "iv"/"sc" does not necessarily mean that the doses differ in any other aspect than the mode of administration. Formulations comprised in an iv-dose and a sc-dose may be identical or they may differ unless otherwise specified, e.g. with respect to volume, weight, amount, concentrations of active ingredients and excipients. The terms "iv-dose" and "sc-dose" are used herein for doses comprising C1-INH unless specified otherwise (e.g., an iv-dose of drug X).

IVIg (intravenous immune globulin) refers to an immunoglobulin that is administered intravenously. In some cases an IVIg may take several hours to administer, and thus, may IVIg be administered over several hours, for example, with breaks periodically as needed for the patient's convenience and comfort.

"Over" as used herein in the context of "over a certain time period" describes a duration of drug administration, e.g. administration of a drug "over 5 days" or "over 10 weeks". In this context the administration of the first dose of the drug marks the first day and administration of the last dose of the drug marks the last day and the duration is given by the number of days counted from the first to the last day, wherein the mode of administration may also characterize the drug in this context. For example, administration of "iv-doses over 5 days" could refer to an intravenous administration of 2 doses on days 1 and 5 or of 3 doses on days 1, 3 and 5 or of 5 doses on days 1, 2, 3, 4, 5 (non-exhaustive list). The same applies accordingly when a drug is given over a certain number of weeks, e.g. the administration of the first dose of the drug marks the first week and administration of the last dose of the drug marks the last week (without need to specify a specific day of the week) and the duration is given by the number of weeks counted from the first to the last week.

"Effective amount" refers to an amount of a drug that is sufficient to achieve a beneficial effect when used for treating a disease or disorder. This may be an amount of a drug that eliminates, alleviates or slows a disease or disorder and/or symptoms related thereto after onset of the disease or disorder or that reduces the risk, prevents or delays the onset of a disease or disorder and/or symptoms related thereto. An effective amount may also refer to an amount effective at dosages and for periods of time necessary to achieve a desired therapeutic or prophylactic effect. The therapeutic effect may be the elimination, alleviation or slowing down of an existing disease or disorder or in the reduction of the severity of it. The prophylactic effect may be the reduction of risk, the prevention or the delay that a subject who does not presently have the disease or disorder will develop the disease or disorder or symptoms and/or signs of the disease or disorder in the future. As used herein the disease or disorder may be AMR.

The "estimated glomerular filtration rate" (eGFR) is determined based on various factors (serum creatinine, age, race and gender) to account for certain differences between subjects. It may be calculated with the MDRD formula. The following IDMS-traceable MDRD formula may be used to determine eGFR of a subject (Levey et al., Ann. Intern. Med. 2009; 150(9): 604-612): $eGFR = 175 \times \left({S}_{cr}^{- 1.154}\right) \times \left({age}^{- 0.203}\right) \times \left(0.742,if female\right) \times \left(1.212,if black\right) .$ Serum creatinine S_{cr} is expressed in mg/dL and the age of subject in years. eGFR is expressed as mL/min per 1.73 m² of body surface area (BSA) of a patient.

The terms "subject" and "patient" refer to a human. A subject or patient as used herein may refer to a human who received a transplant (a "transplant recipient" or a "recipient"), such as an "allograft transplant recipient" or an "organ transplant recipient." The subject or patient or recipient as used herein may also refer to a human who is scheduled to receive a transplant and/or will receive a transplant, e.g. will receive an organ transplant or allograft transplant within a month or week.

As used herein, an "allograft" is a transplant of cells, tissue, or one or more organs from a genetically non-identical donor of the same species. A "transplant" refers to the transplantation of an organ, tissue or cells. An "organ transplant" refers to the transplantation of a complete or essentially complete organ. In some embodiments a transplant recipient may receive an allograft. In other embodiments, a transplant recipient may receive a "xenograft," which is a transplant of cells, tissue, or one or more organs from a donor of a different species (e.g. a pig or other mammal). In some embodiments the transplant is a solid organ, e.g. a kidney.

"Standard of Care" (SOC) as used herein refers to a treatment with one or more immunomodulatory/immunosuppressive agents. In some embodiments the immunomodulatory/immunosuppressive agents are selected from intravenous immunoglobulin (IVlg), anti-CD20 antibodies (e.g. rituximab), mycophenolate mofetil (MMF), cyclosporine, corticosteroids (e.g. methylprednisolone, prednisone), calcineurin inhibitors (e.g. tacrolimus), bortezomib and antithymocyte globulin (ATG). In some cases they inhibit B cell or T cell activity (e.g. by reducing division or signaling to cells or depletion of cells). In addition, SOC treatment may include plasmapheresis and/or immunoadsorption. SOC treatment may comprise administration of IVIg. SOC treatment may comprise administration of IVIg with or without plasmapheresis and/or rituximab. For example, SOC treatment may be administration of IVIg optionally with plasmapheresis as needed and optionally rituximab. In some cases, one or more corticosteroids and calcineurin inhibitors may be administered either with the IVIg or instead of the IVIg. In some cases the amount of IVIg administered is lower with plasmapheresis (e.g. 100 mg/kg) than without plasmapheresis (e.g. 1 or 2 g/kg). The term SOC treatment does not comprise administration of C1-INH, but it is possible to administer C1-INH according to the dosing regimen described herein in parallel to SOC treatment unless specified otherwise. For example, in some embodiments a patient may be treated with SOC without administration of C1-INH, but if SOC treatment alone does not succeed, SOC treatment may be continued in addition to administration of C1-INH in parallel in accordance with the dosing regimen described herein as add-on therapy. In other situations, C1-INH may be administered as an adjunct to SOC treatment.

The terms "treatment", "treat" or "treating" as used herein may refer to administering a drug (i) to reduce or eliminate at least one symptom or sign of a specified disease or disorder or (ii) to slow down, alleviate or stop the progression of a specified disease or disorder or (iii) to delay, hinder or stop the development of a specified disease or disorder. Accordingly, the terms "treatment", "treat" or "treating" may refer to therapeutic treatment as well as prophylactic treatment (also called prevention) of a disease or disorder with a drug. "Therapeutic treatment" and "therapeutic treating" refers to the treatment of an existing disease or disorder, i.e. treatment after the onset of the disease or disorder and its aim is to eliminate, alleviate or slow down the existing disease or disorder or to reduce the severity of it. In contrast, "prophylactic treatment", "prevention", "prevent" or "preventing" refers to a type of treatment for the disease or disorder intended to reduce the risk, prevent or delay that a subject who does not presently have the disease or disorder will develop it or symptoms or signs of it in the future, in particular after an event like a transplantation before onset of AMR. A "chronic treatment" as used herein refers to a treatment with a relatively long duration of 2 or more months and may last even years.

One "unit" ("U") of C1-INH is equivalent to the C1-INH activity in 1 mL of fresh citrated plasma of healthy donors. C1-INH may also be determined in "international units" ("IU"). These international units are based on the current World Health Organization (WHO) standard for C1-INH concentrates (NIBSC code: 08/256), which was calibrated in an international collaborative study using normal local human plasma pools. U and IU are equivalent and used interchangeably herein.

For the above defined terms grammatical variations thereof have not been discussed explicitly in all cases, but it should be understood that definitions apply accordingly for any grammatical variations even if they are not explicitly mentioned. Furthermore, the above defined terms may be more fully defined by reference to the specification in its entirety.

### TREATMENT OF AMR

The present disclosure includes C1-INH for use in methods of treating antibody-mediated rejection in a transplant recipient subject. Some methods herein comprise administering C1-INH to the subject according to a schedule with the following steps in this order: (a) intravenously administering one or more iv-doses of C1-INH, (b) subcutaneously administering at least 10 sc-doses of C1-INH over several weeks, such as at least 10 weeks, wherein each week at least one sc-dose is administered. In some embodiments, the iv-doses of C1-INH are 40 to 240 IU/kg, such as 40 to 180 IU/kg, such as 40 to 120 IU/kg, such as 40 to 90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg. In some embodiments, the iv-doses are 40 to 180 IU/kg. In some embodiments, the iv-doses are 40 to 90 IU/kg. In some embodiments, the sc-doses of C1-INH are 40 to 240 IU/kg, such as 40-120 IU/kg, such as 40-90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg. In some embodiments, the sc-doses are 40 to 180 IU/kg. In some embodiments, both the iv-doses and the sc-doses are 40 to 180 IU/kg. In some embodiments, the sc-doses are 40 to 90 IU/kg. In some embodiments, both the iv-doses and the sc-doses are 40 to 90 IU/kg.

The present disclosure also includes C1-INH for use in methods of treating antibody-mediated rejection in a transplant recipient subject comprising administering C1-INH to the subject according to a schedule with the following steps in this order: (a) intravenously administering 3 to 10 iv-doses over 4 to 16 days, (b) subcutaneously administering at least 20 sc-doses C1-INH about twice or three times weekly over 10 or more weeks. In some embodiments, the iv-doses and/or the sc-doses are at 40 to 120 IU/kg.

The present disclosure also includes C1-INH for use in methods of treating antibody-mediated rejection in a transplant recipient subject comprising administering C1-INH to the subject according to a schedule with the following steps in this order: (a) intravenously administering 3 to 10 iv-doses each with 2,500 to 8,500 IU C1-INH, (b) subcutaneously administering at least 20 sc-doses each comprising 2,500 to 8,500 IU C1-INH about twice or three times weekly over 10 or more weeks.

The present disclosure further includes C1-INH for use in methods of treating antibody-mediated rejection in a transplant recipient subject comprising administering C1-INH to the subject according to a schedule with the following steps in this order: (a) intravenously administering a total amount of 7,000 to 36,000 IU C1-INH in divided iv-doses over 2 to 21 days, (b) subcutaneously administering an amount of at least 50,000 IU C1-INH in divided sc-doses over at least 10 weeks, wherein each week at least one sc-dose is administered.

Further methods comprise treating antibody-mediated rejection in a transplant recipient comprising subcutaneously administering C1-INH over at least 10 weeks, wherein each week at least one sc-dose is administered. In some of the methods C1-INH is administered exclusively subcutaneously.

Further exemplary embodiments include C1-INH for use in methods of treating antibody-mediated rejection in a transplant recipient subject comprising administering C1-INH only according to step (b) by subcutaneously administering at least 10 sc-doses of 40 to 200 IU/kg, such as 40 to 180 IU/kg, such as 40-120 IU/kg, such as 40-90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg C1-INH to the subject over at least 10 weeks, wherein each week at least one sc-dose is administered. In this alternative method of treatment there is not step (a) and all C1-INH is administered subcutaneously.

The disclosure also encompasses C1-INH for use in methods of improving kidney function or eGFR in a subject who received a renal allograft (e.g. kidney transplant) comprising administering C1-INH to the subject according to a schedule with the following steps: (a) intravenously administering one or more iv-doses of 40 to 200 IU/kg, such as 40 to 180 IU/kg, such as 40 to 120 IU/kg, such as 40 to 90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg C1-INH, (b) subcutaneously administering at least 10 sc-doses of 40 to 200 IU/kg, such as 40 to 180 IU/kg, such as 40-120 IU/kg, such as 40-90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg IU/kg C1-INH over at least 10 weeks, wherein each week at least one sc-dose is administered.

The disclosure further encompasses C1-INH for use in methods of treating antibody-mediated rejection in a transplant recipient comprising administering C1-INH subcutaneously, optionally at doses of 40 to 200 IU/kg, such as 40 to 180 IU/kg, such as 40-120 IU/kg, such as 40-90 IU/kg, such as 60 to 200 IU/kg, such as 60 to 180 IU/kg, such as 90 to 200 IU/kg, such as 90 to 180 IU/kg, such as 120 to 200 IU/kg, or such as 120 to 180 IU/kg. In some embodiments, the C1-INH is given at least once each week for at least 10 weeks. In some embodiments, the C1-INH is given as an adjunct to an SOC treatment. In some embodiments, the C1-INH is given in addition to IVIg with or without plasmapheresis and with or without rituximab. In some embodiments, polypeptide comprises an XTEN sequence. In some embodiments the XTEN is fused to the C-terminus of the same or essentially the same amino acid sequence as for C1 inhibitor naturally occurring in humans. In some embodiments the half-life enhancing polypeptide is selected from the group consisting of albumin, afamin, alpha-fetoprotein, vitamin D binding protein, human albumin, XTEN sequence, C-terminal peptide (CTP), an immunoglobulin, and an Fc of an IgG. For example, the half-life enhancing polypeptide may be albumin or a variant thereof. In some embodiments the half-life enhancing polypeptide comprises an albumin or a variant thereof. In some embodiments an albumin variant includes part or all of specific domains of human albumin (HA). An albumin variant may include an amino acid substitution, deletion, or addition, either conservative or non-conservative substitution, wherein such changes do not substantially alter the active site, or active domain, which confers the therapeutic activities of the half-life enhancing polypeptides. These variants may share identity of about 70%, or about 75%, or about 80%, or about 85%, or about 90%, or about 91%, or about 92%, or about 93%, or about 94%, or about 95%, or about 96%, or about 97%, or about 98% or about 99% from a human albumin (HA) sequence. In one example, an albumin variant is a fragment. In one example, the albumin variant comprises at least one domain of albumin and/or fragments of those domains. In some embodiments the half-life enhancing polypeptide is an immunoglobulin (Ig) or a functional fragment or a variant thereof, such as an Fc region or one or more Ig constant domains. In one example, the Ig comprises an Fc region or portions of the immunoglobulin constant domain(s). The constant region may be that of an IgM, IgG, IgD, IgA, or IgE immunoglobulin. In one example, the therapeutic polypeptide portion is connected to the Ig via the hinge region of the antibody or a peptide linker, which may be cleavable. Methods for the fusion of therapeutic proteins to immunoglobulin constant regions to extend the therapeutic protein's half-life *in vivo* are known in the art and are described in e.g., US 2004/0087778, WO 2005/001025, WO 2005/063808, WO 2003/076567, WO 2005/000892, WO 2004/101740, US 6,403,077. In one example, the half-life enhancing polypeptide is an immunoglobulin region. For example, the immunoglobulin region is an Fc domain, or an Fc fragment of immunoglobulins, and/or variants thereof. In some embodiments the amino acid sequence of C1 inhibitor as naturally occurring in humans is fused to Fc domains or portions of immunoglobulin constant regions as HLEPs. In some embodiments the above described fusion proteins are prepared as recombinant molecules expressed in prokaryotic or eukaryotic host cells. For example, the fusion proteins may be prepared in bacteria, or yeast, or plant, or animal (including insect) or human cell lines or in transgenic animals. Methods of the expression of fusion proteins in prokaryotic or eukaryotic cells are known in the art and are described in e.g., WO 2008/098720. Some variants of C1-INH based fusion proteins are provided in WO2016/070156. In some embodiments the fusion proteins have a half-life which is at least as long as the half-life for plasma derived C1-INH when administered intravenously. In some embodiments the fusion proteins have a half-life which is longer, but not more than twice as long as the half-life for plasma derived C1-INH when administered intravenously.

In some embodiments comprising (a) intravenous and (b) subcutaneous administration, at least 2 or at least 3 iv-doses of C1-INH are administered in step (a). In some embodiments less than 15 or less than 10 iv-doses or less than 5 iv-doses of C1-INH are administered in step (a). In some embodiments 1, 2, 3, 4, 5, 6, 7, 8, 9 or 10 iv-doses of C1-INH are administered in step (a).

In some embodiments at least 2 iv-doses of C1-INH are administered intravenously over 1, 2, 3, 4, 5, 6, 7, 8, 9, 10, 11, 12, 13, 14, 15, 16, 17, 18, 19, 20, 21, 22 or 23 days in step (a). In some embodiments the iv-doses of C1-INH are administered over less than 22, less than 17, or less than 14 days in step (a). In some embodiments the iv-doses are administered over at least 2 days or over at least 3 days in step (a).

In some embodiments at least 2 iv-doses of C1-INH are administered over 2 to 21 days in step (a). In some embodiments step (a) comprises intravenously administering at least 2 iv-doses of 40 to 180 IU/kg C1-INH over 2 to 21 days. In some embodiments at least 3 iv-doses of C1-INH are administered over 2 to 21 days. In some embodiments 3 to 10 iv-doses of C1-INH are administered over 2 to 21 days in step (a). In some embodiments 3 to 5 iv-doses of C1-INH are administered over 2 to 21 days in step (a).

In some embodiments at least 2 iv-doses of C1-INH are administered over 4 to 16 days in step (a). In some embodiments 3 to 10 iv-doses of C1-INH are administered over 4 to 16 days in step (a). Accordingly, in some embodiments step (a) comprises intravenously administering 3 to 10 iv-doses of 40 to 180 IU/kg C1-INH over 4 to 16 days. In some embodiments 3 to 5 iv-doses of C1-INH are administered over 4 to 16 days in step (a). In some embodiments 3 to 10 iv-doses of C1-INH are administered over 7 to 13 days in step (a).

In some embodiments at least 2 iv-doses of C1-INH are administered over 7 to 13 days in step (a). In some embodiments 3 to 5 iv-doses of C1-INH are administered over 7 to 13 days in step (a). Accordingly, in some embodiments step (a) comprises intravenously administering 3 to 5 iv-doses of 40 to 180 IU/kg C1-INH over 7 to 13 days. In some embodiments 3 iv-doses of C1-INH are administered over 13 days in step (a). In some embodiments 4 iv-doses of C1-INH are administered over 13 days in step (a). In some embodiments 5 iv-doses of C1-INH are administered over 13 days in step (a). In some embodiments 3 iv-doses of C1-INH are administered over 10 days in step (a). In some embodiments 4 iv-doses of C1-INH are administered over 10 days in step (a). In some embodiments 5 iv-doses of C1-INH are administered over 10 days in step (a). In some embodiments 5 iv-doses of C1-INH are administered over 7 days in step (a). In some embodiments 4 iv-doses of C1-INH are administered over 7 days in step (a). In some embodiments 3 iv-doses of C1-INH are administered over 7 days in step (a).

In some embodiments the iv-doses are administered every second, third or fourth day in step (a). In some embodiments step (a) comprises intravenously administering at least 2 iv-doses of 40 to 180 IU/kg C1-INH to the subject, wherein the iv-doses are administered every second or third or fourth day.

In some embodiments between each administration of an iv-dose of C1-INH there is at least one day without administration of an iv-dose of C1-INH in step (a). In some embodiments step (a) comprises intravenously administering at least 2 iv-doses of 40 to 180 IU/kg C1-INH to the subject, wherein between each administration of one of the iv-doses of C1-INH there is at least one day without administration of one of the iv-doses of C1-INH. In some embodiments between each administration of one of the iv-doses of C1-INH there are at least 2 days without administration of one of the iv-doses of C1-INH. For example, administration could be on days 1, 4, 13 and this would mean at least 2 days without administration of C1-INH are between day 1 and day 4 and between day 4 and day 13.

In some embodiments a total amount of 7,000 to 36,000 IU C1-INH is administered intravenously in step (a). In some embodiments a total amount of less than 40,000 IU C1-INH or less than 30,000 IU C1-INH is administered in step (a). In some embodiments a total amount of more than 5,000 IU C1-INH or more than 10,000 IU C1-INH is administered in step (a). In some embodiments a total amount of 10,000 to 30,000 IU C1-INH is administered intravenously in step (a). In some embodiments a total amount of 13,000 to 25,000 IU C1-INH is administered intravenously in step (a).

In some embodiments the combined amount of C1-INH administered in steps (a) and (b) is an effective amount for treating antibody-mediated rejection when administered intravenously and subcutaneously as described herein. In some embodiments the amount of C1-INH administered in step (b) is an effective amount for treating antibody-mediated rejection.

In some embodiments an amount of 4,000 to 15,000 IU C1-INH is administered subcutaneously per week. In an embodiment, subcutaneous administration comprises subcutaneously administering at least 10 sc-doses of 40 to 180 IU/kg C1-INH to the subject over at least 10 weeks, wherein an amount of 4,000 to 15,000 IU C1-INH is administered subcutaneously every week. In some embodiments an amount of 4,000 to 10,000 IU C1-INH is administered each week during subcutaneous administration. In some embodiments an amount of 5,000 to 9,000 IU C1-INH is administered each week during subcutaneous administration.

In some embodiments an amount of 80 to 360 IU/kg C1-INH is administered each week during subcutaneous administration, e.g. divided into 2 weekly sc-doses of 40 to 180 IU/kg. In some embodiments an amount of 80 to 240 IU/kg C1-INH is administered each week during subcutaneous administration, e.g. divided into 2 weekly sc-doses of 40 to 120 IU/kg. In some embodiments an amount of 80 to 180 IU/kg C1-INH is administered each week during subcutaneous administration, e.g. divided into 2 weekly sc-doses of 40 to 90 IU/kg. In some embodiments an amount of 80 to 160 IU/kg C1-INH is administered each week during subcutaneous administration, e.g. divided into 2 weekly sc-doses of 40 to 80 IU/kg. In some embodiments an amount of 80 to 140 IU/kg C1-INH is administered each week during subcutaneous administration, e.g. divided into 2 weekly sc-doses of 40 to 70 IU/kg. In some embodiments an amount of 100 to 160 IU/kg C1-INH is administered each week during subcutaneous administration, e.g. divided into 2 weekly sc-doses of 50 to 80 IU/kg. In some embodiments an amount of 100 to 140 IU/kg C1-INH is administered each week during subcutaneous administration, e.g. divided into 2 weekly sc-doses of 50 to 70 IU/kg.

In some embodiments each iv-dose and/or each sc-dose contains 40 to 240 IU/kg C1-INH. In some embodiments each iv-dose and/or each sc-dose contains 40 to 180 IU/kg C1-INH. In some embodiments each iv-dose and/or each sc-dose contains 40 to 120 IU/kg C1-INH. In some embodiments each iv-dose and/or each sc-dose contains 40 to 100 IU/kg C1-INH. In some embodiments each iv-dose and/or each sc-dose contains 40 to 90 IU/kg C1-INH. In some embodiments each iv-dose and/or each sc-dose contains 40 to 80 IU/kg C1-INH. In some embodiments each iv-dose and/or each sc-dose contains 50 to 70 IU/kg C1-INH. In some embodiments each iv-dose and/or each sc-dose contains 40, 45, 50, 55, 65, 70, 75, 80, 85, 90, 100, 110 or 120 IU/kg C1-INH. In some embodiments each iv-dose and/or each sc-dose contains 60 IU/kg C1-INH.

In some embodiments each iv-dose and/or each sc-dose contains 2,500 to 8,500 IU/kg C1-INH. In some embodiments each iv-dose and/or each sc-dose contains 2,500 to 5,500 IU/kg C1-INH.

In some embodiments after completion of step (b) the subject does not receive C1-INH for at least 1 month, for at least 2 months, for at least 3 months or for at least 6 months. In some embodiments after completion of step (b) the subject does not receive C1-INH as part of the herein disclosed course of treatment anymore.

In some embodiments all C1-INH is administered in steps (a) and (b) and the method comprises no administration of C1-INH apart from this. In some embodiments all intravenously administered C1-INH is administered in step (a) and (b) and all subcutaneously administered C1-INH is administered in step (b).

In some embodiments C1-INH comprises an additional administration of C1-INH in a step (c) following step (b). This step (c) may also be called a retreatment step. In some embodiments the step (c) is a repetition of step (b). In some embodiments the optional step (c) has the same amount of C1-INH per sc-dose, the same frequency of administration and the same mode of subcutaneous administration as step (b), but may comprise administering a higher or lower amount of C1-INH over a shorter or longer time period than step (b). In some embodiments the step (c) may have a higher or lower amount of C1-INH per sc-dose than step (b). Retreatment may be helpful in case of a relapse soon after discontinuation of C1-INH administration. In some embodiments there is at least 1 week or at least 1 month without administration of C1-INH between steps (b) and (c). In some embodiments step (b) may comprise an optional retreatment phase.

In some embodiments the sc-doses of C1-INH are administered about once weekly, about twice weekly, about three times weekly or about four times weekly. In some embodiments the sc-doses are administered about twice weekly in step (b). In some embodiments the sc-doses are administered about three times weekly in step (b). In some embodiments the sc-doses are administered about once weekly in step (b). In some embodiments the sc-doses are administered twice weekly in step (b). In some embodiments the sc-doses are administered three times weekly in step (b). In some embodiments the sc-doses are administered once weekly in step (b).

In some embodiments at least 5, at least 10, at least 15, at least 20 or at least 25 of the sc-doses comprising C1-INH are administered during subcutaneous administration. In some embodiments at least 20 sc-doses of C1-INH are administered during subcutaneous administration. In some embodiments where both intravenous and subcutaneous dosing is used in steps (a) and (b), at least 2 times as many, at least 3 times as many, at least 4 times as many or at least 5 times as many sc-doses are administered in step (b) as iv-doses are administered in step (a). In some embodiments at least 2 as much, at least 3 times as much, at least 4 times as much or at least 5 times as much C1-INH is administered subcutaneously in step (b) as is administered intravenously in step (a).

In some embodiments the sc-doses are administered over a period of time that is at least 2 times as long, at least 3 times as long, at least 4 times as long, at least 5 times as long, at least 6 times as long, at least 7 times as long, at least 8 times as long, at least 9 times as long or at least 10 times as long as a period of time for administration of the iv-doses. In some embodiments the sc-doses are administered over a period of time that is at least 5 times as long as a period of time for administration of the iv-doses. A duration for administering C1-INH intravenously in step (a) may also be referred to as a first time period and a duration for administering C1-INH subcutaneously in step (b) as a second time period. Accordingly, in some embodiments the second time period is at least 2 times as long, at least 3 times as long, at least 4 times as long, at least 5 times as long, at least 6 times as long, at least 7 times as long, at least 8 times as long, at least 9 times as long or at least 10 times as long as the first time period. In an embodiment the method comprises the following steps: (a) intravenously administering one or more iv-doses of 40 to 180 IU/kg C1-INH over a first time period, (b) subcutaneously administering at least 10 sc-doses of 40 to 180 IU/kg C1-INH over a second time period of at least 10 weeks, wherein the second time period is at least at least 5 times as long as the first time period.

In some embodiments at least twice as much C1-INH is administered subcutaneously as is administered intravenously. In some embodiments an amount of C1-INH is administered in step (b), which is at least twice as high as an amount of C1-INH administered in step (a). In some further embodiments the amount of C1-INH administered subcutaneously in step (b) is at least twice, at least three or at least four times higher than the amount of C1-INH administered intravenously in step (a).

In some embodiments more than 50,000 IU C1-INH is administered in total in step (a) and (b), or during the overall dosing regimen. In some embodiments more than 100,000 IU C1-INH is administered in total. In some embodiments more than 150,000 IU C1-INH is administered in total. In some embodiments more than 200,000 IU C1-INH is administered in total.

In some embodiments with both intravenous (step (a)) and subcutaneous (step (b)) dosing, more than 50,000 IU C1-INH is administered subcutaneously in step (b). In some embodiments more than 100,000 IU C1-INH is administered subcutaneously in step (b). In some embodiments more than 150,000 IU C1-INH is administered subcutaneously in step (b). In some embodiments more than 200,000 IU C1-INH is administered subcutaneously in step (b). In some embodiments a total amount of 7,000 to 36,000 IU C1-INH is administered intravenously in step (a) and an amount of more than 50,000 IU C1-INH is administered subcutaneously in step (b). In some embodiments less than 1,000,000 IU C1-INH is administered subcutaneously in step (b). In some embodiments less than 750,000 IU C1-INH is administered subcutaneously in step (b). In some embodiments less than 500,000 IU C1-INH is administered subcutaneously in step (b). In some embodiments there is no upper limit for the amount of C1-INH administered subcutaneously in step (b) and subcutaneous administration is continued as long as possible e.g. until death of subject or allograft loss.

In some embodiments the concentration of C1-INH of the formulation administered ranges from 50 to 1,500 IU/mL, in particular after reconstitution. In some embodiments the concentration of C1-INH during administration ranges from 100 to 1,000 IU/mL, in particular from 200 to 800 IU/mL or from 400 to 600 IU/mL. For example, the C1-INH formulation administered may have a concentration of 500 IU/mL C1-INH after reconstitution.

In some embodiments the formulations for the iv-doses and sc-doses are identical. In some embodiments the iv-doses and sc-doses are different, e.g. with respect to the concentration of C1-INH. In some embodiments the formulation for sc-doses has a higher concentration than the formulation for iv-doses.

In some embodiments each iv-dose contains the same amount of C1-INH, e.g. each iv-dose may comprise 60 IU/kg C1-INH. In some embodiments each sc-dose contains the same amount of C1-INH, e.g. each sc-dose may comprise 60 IU/kg C1-INH. In some embodiments a first iv-dose may comprise more C1-INH that subsequent iv-doses, e.g. a loading iv-dose of 120 IU/kg C1-INH followed by iv-doses of 60 IU/kg C1-INH each.

In some embodiments each iv-dose comprises the same amount of C1-INH as each sc-dose. For example, the doses for intravenous and subcutaneous injection may both be 60 IU/kg C1-INH per dose. In some embodiments the amount of C1-INH of each iv-dose is the same as the amount of C1-INH of each sc-dose and the formulations for iv-doses and sc-doses are identical as well, i.e. for such embodiments the only difference between iv-dose and sc-dose is the mode of administration.

In some embodiments the subject is an allograft recipient. In some embodiments the subject is a xenograft recipient. In some embodiments, the subject is a transplant recipient. In some embodiments the allograft is selected from kidney (i.e. renal allograft), heart, liver, lung, intestine or pancreas. In some embodiments the allograft is heart, lung or kidney. In some embodiments the subject is a renal allograft recipient. In some embodiments the subject has received the allograft at least one week or at least two weeks or at least three weeks or at least one month prior to administering C1-INH according to step (a). In some embodiments the subject has been diagnosed with AMR or is at risk of AMR.

In some embodiments the subject is a patient scheduled to receive an allograft in the future, in particular an allograft selected from kidney, heart, liver, lung, intestine or pancreas. For example, the subject may be a patient for whom a renal transplant surgery is planned within the next week(s) (e.g., one to four weeks) or day(s). It may be beneficial to initiate treatment according to step (a) prior to the surgery to prevent occurrence of AMR after surgery. In some embodiments the subject is at risk of future AMR in view of a planned transplant surgery.

In some embodiments treatment of the subject with the C1-INH is initiated more than three months after transplantation.

In some embodiments the transplantation is a surgery in which the subject receives a renal allograft.

In some embodiments the method is for therapeutic treating of antibody-mediated rejection. In some of those embodiments the method is for therapeutic treating antibody-mediated rejection in a renal allograft recipient. In an alternative embodiment the method is for preventing antibody-mediated rejection, for example, by administering the C1-INH prior to onset of symptoms of AMR after transplantation or, alternatively, administering the C1-INH before the transplantation.

In some embodiments the method is for treating refractory antibody-mediated rejection, in particular for therapeutic treating refractory antibody-mediated rejection. Treatment of refractory antibody-mediated rejection is particularly difficult because chances of success are generally lower in view of a previous non-successful SOC treatment.

In some embodiments the method is for treating active antibody-mediated rejection. In some embodiments the method is for treating chronic active antibody-mediated rejection.

In some embodiments the recipient suffers from antibody-mediated rejection after transplantation. In some embodiments the recipient has active antibody-mediated rejection. In some embodiments the recipient has chronic active antibody-mediated rejection.

In some embodiments the method is for chronic treating of AMR. Active AMR as well as chronic active AMR may benefit from chronic treatment. In some embodiments the method is for chronic treating of active AMR. In some embodiments the method is for chronic treating of chronic active AMR. In some embodiments the method is for chronic treating of refractory AMR. In chronic treatment, administration according to step (b) may continue for months or years or continue for as long as possible, for example, until death of the subject or organ failure. For example, long-term subcutaneous administration of C1-INH may have a beneficial effect for graft function.

In some embodiments IVIg is administered in addition to administration of the C1-INH as described herein. In some embodiments with intravenous and subcutaneous dosing in steps (a) and (b), IVIg is administered in step (a) in addition to the herein described intravenous administration of C1-INH. In some embodiments IVIg is administered in addition to subcutaneous administration of C1-INH in step (b). In some embodiments IVIg is administered in step (a) as well as in step (b) in addition to the C1-INH. In some embodiments in which C1-INH is administered subcutaneously only, IVIg is administered along with the subcutaneous C1-INH administration. In other cases, C1-INH is administered after at least one administration of IVIg. In some embodiments IVIg is administered as infusions every 3 to 5 weeks. In some embodiments each infusion comprises 0.05 to 3 g/kg IVIg. In some embodiments each infusion comprises 0.1 to 2 g/kg IVIg. In some embodiments at least one infusion comprises 50 mg/kg to 500 mg/kg IVIg. In some embodiments at least one infusion comprises 100 mg/kg to 200 mg/kg IVIg. In some embodiments at least one infusion comprises 1 g/kg to 3 g/kg IVIg. In some embodiments the infusion may be administered within 12 to 96 hours, in particular within 24 to 48 hours. In some embodiments IVIg is administered over 12 to 96 hours, in particular within 24 to 48 hours, in several infusions to allow for breaks, e.g. to avoid an infusion during sleep time of the subject. In some embodiments IVIg is administered several days, wherein each day at least one infusion is administered over 2-6 hours. When IVIg is administered in addition to administration of C1-INH, this does not necessarily mean that both drugs are administered at the same time. For example, they may be administered sequentially or concurrently. For example, IVIg may be administered at any time between two doses of C1-INH administration. However, when IVIg is administered in addition to administration of C1-INH, both therapies are implemented in parallel, i.e. the subject will receive both drugs during the same treatment period, e.g. the treatment period of step (a) and/or (b). In some embodiments, the frequency of administration may be lower for IVIg than for C1-INH.

In some further embodiments standard of care treatment without administration of C1-INH is implemented prior to the start of the C1-INH treatments described herein. This applies in particular to cases where the treatment in accordance with the claimed invention is initiated because SOC treatment alone is not successful. In some further embodiments SOC treatment is afterwards continued in parallel to treatment with C1-INH, for instance according to steps (a) and/or (b). In some embodiments the administration of C1-INH in accordance with steps (a) and (b) is an add-on therapy to SOC for AMR that is refractory to SOC treatment alone. In some embodiments SOC without C1-INH may be a first line treatment and subsequent treatment with C1-INH as well as SOC represents a second line treatment, e.g. starting SOC earlier and continuation of SOC treatment in parallel when administering C1-INH according to steps (a) and (b). In some embodiments SOC without C1-INH may be a first line treatment and subsequent treatment with C1-INH without SOC represents the second line treatment.

In some embodiments with both intravenous and subcutaneous administration, step (b) is after step (a). In some embodiments there is a time window (without any administration of C1-INH) between the last administration of an iv-dose according to step (a) and the first administration of a sc-dose according to step (b). In some embodiments the time window is 12 to 96 hours. In some embodiments the time window is 18 to 72 hours. In some embodiments the time window is 24 to 48 hours.

In some embodiments iv-doses are administered by healthcare professionals.

In some embodiments at least some of the sc-doses are self-administered by the subject in step (b). In some embodiments all sc-doses are self-administered and in other embodiments only some or none of them are. Those sc-doses that are not self-administered may for example be administered by healthcare professionals. It has been estimated that non-adherence can be detected in approximately 50% of failing grafts (Moreso et al., Transplant Research and Risk Management 2015; 7: 27-34) and it further has been reported that concerns about non-adherence were recorded 10 times more frequently in patients whose graft subsequently failed than in those whose grafts have not failed (Sellarés al., Am. J. Transplant. 2012; 12(2): 388-399). The option of self-administration may increase adherence rate for subjects and increase allograft survival.

### EXAMPLES

The examples discussed below are intended to be purely exemplary of the invention and should not be considered to limit the invention in any way. Efforts have been made to ensure accuracy with respect to numbers used (for example, amounts, durations etc.) but some experimental errors and deviations should be accounted for.

### EXAMPLE 1: Study to evaluate the efficacy and safety of human plasma-derived C1 esterase inhibitor as add-on to standard of care for the treatment of antibody mediated rejection in adult renal transplant recipients over 12 weeks.

A study to evaluate the efficacy and safety of human plasma-derived C1 inhibitor as add-on to standard of care for the treatment of antibody mediated rejection in adult renal transplant recipients was conducted.

### Investigational Product

pdC1-INH (500 IU/mL) was manufactured according to ICH Good Manufacturing Practice guidelines and local regulatory requirements and provided as lyophilized powder (1,500 IU C1-INH per single-use vial). Before use, each vial of C1-INH was reconstituted with 3 mL water for injection. After reconstitution, C1-INH was available at a concentration of 500 IU/mL for intravenous or subcutaneous administration. 60 IU/kg C1-INH is equivalent to a volume of 0.12 mL/kg. The actual dose of C1-INH was rounded down to the nearest 500 IU, as this corresponds with a practical volume of 1 mL of C1-INH.

### Eligibility Criteria

The study population was selected on the basis of the inclusion and exclusion criteria described hereinafter. Eligible patients were at least 18 years old and were recipients of a kidney transplant that met criteria for acute AMR according to Banff 2015 (Loupy et al., Am. J. Transplant. 2017; 17(1): 28-41). This was confirmed by histologic evidence of acute tissue inflammation with presence of neutrophils and/or monocytes (g > 0, v > 0, and/or ptc > 0), and C4d positive or, if C4d negative, then g + ptc ≥ 2 (g = Banff lesion score for glomerulitis; v = Banff lesion score for intimal arteritis; ptc = Banff lesion score for peritubular capillaritis; cf. Banff criteria for details). Patients that met Banff criteria for active AMR as well as for chronic active AMR were included as well. In accordance with Banff 2015, only patients that showed evidence of at least one donor-specific antibody (DSA) were included. Other eligibility criteria were a steady-state, post-transplant eGFR ≥ 40 mL/min/1.73 m² within 60 days of transplant or a 50% increase in urine output with a 50% decrease in serum creatinine over the first 7 days post-transplant in subjects with slow or delayed graft function. Recipients of en bloc kidney transplants were excluded. Further exclusion criteria were hepatitis C; an active bacterial or fungal infection; an ongoing dialysis > 2 weeks; a known congenital bleeding or coagulopathy disorder; current cancer or a history of cancer; female subjects who were pregnant or breast feeding; male or female subjects who were unwilling to use contraception or not surgically sterile.

Administration of other complement inhibitors, proteasome inhibitors, immune suppressants that were not licensed for clinical use or any other investigational drugs was not permitted during this study. Subjects were not enrolled into the study if they received any of these prohibited therapies.

Only patients were eligible for treatment with C1-INH that did not respond to standard of care (SOC) as a first line treatment for AMR. For this study SOC treatment was specified as either a) IVIg ≥ 100 mg/kg and plasmapheresis; or b) IVIg > 1 gram/kg without plasmapheresis. Unresponsive to SOC was defined as no improvement in renal function (e.g. eGFR) as determined by the treating physician in ≥7 days for SOC regimen a) or ≤45 days for SOC regimen b). Rituximab use was permitted as an optional component of SOC. Use of C1-INH was not permitted for this first line treatment. Those patients that were refractory to the above specified standard of care treatment were subsequently treated with C1-INH as described below.

### Study Design and Interventions

The study was conducted at multiple sites globally. 63 patients met the above eligibility criteria and 61 began initial treatment with C1-INH, of whom 47 were subsequently treated with C1-INH as described herein and had completed treatment (7 withdrew from the study prior to completion of the study, and 7 had not yet completed the study).

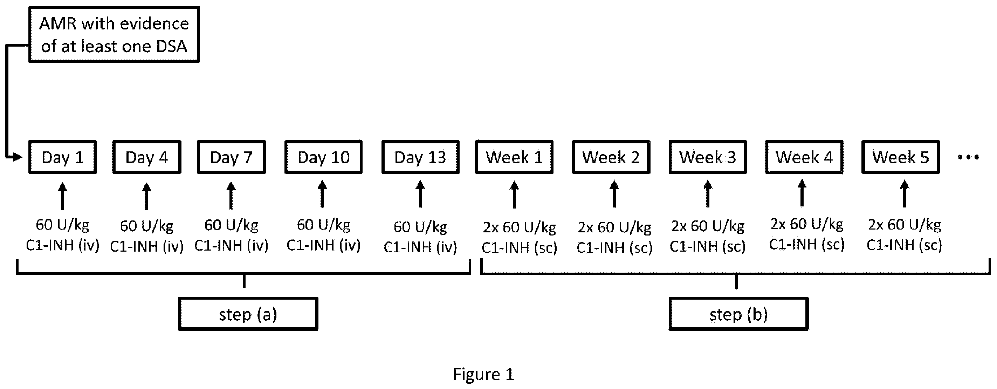

To each of these 47 patients 5 doses of C1-INH (60 IU/kg) were administered intravenously over 13 days (administrations scheduled for Days 1, 4, 7, 10, 13). Thereafter, C1-INH (60 IU/kg twice weekly) was administered subcutaneously for an additional 10 weeks (12 weeks in total). During these 12 weeks of treatment intravenous immunoglobulin (IVIg) was administered to all patients at 2 grams/kg per infusion every 4 weeks (IVIg at weeks 1, 4, 8 and 12). Each administration of IVIg took 2 to 5 days (2-4 hour daily infusions with breaks during and/or between infusions). Figure 1 shows the treatment schedule for C1-INH (SOC is not shown). Plasmapheresis could be performed if a subject had high DSA. High DSA was defined as at least one DSA (to HLA class I and/or class II) with a normalized MFI value ≥ 5000. If a scheduled dose of C1-INH was the same day as plasmapheresis, it was administered after plasmapheresis. If plasmapheresis occurred during a scheduled IVIg administration, administration of IVIg was postponed until the plasmapheresis session was completed.

### Results

Median age of the recipients was 41 years (range 18 to 71 years). Most recipients (56%) were male. Racial distribution was 63% white, 18% black, 8% Asian, and 11% multiple or other. Median body weight was 72 kg (range 26 to 162 kg) and median body mass index was 25.9. Most donors were deceased (66%), of which approximately 49% were donors after brain death, approximately 16% were donors after circulatory death and donor type information was missing for 34%; brain dead donors were standard criteria donors. Median donor age was 46 years (range 9 to 74 years). Donor sex was distributed evenly (48% male, 48% female, 5% data missing). Median KDPI score was 53%.

Of the recipients where such data were available, approximately two thirds showed C4d deposition. 75% of patients had DeNovo DSA and 21% patients had DSA prior to transplant. The median time from most recent transplantation to AMR diagnosis was 148 days. The median time from diagnosis of AMR to the start of treatment with C1-INH was 43 days.
eGFR values changes during the 12 weeks' C1-INH treatment period were compared and are shown below:

**Table 1: eGFR development during treatment**

| | Baseline eGFR (mL/min/1.73 m²) | Average of Week 11 and 12 (mL/min/1.73 m²) | Change (mL/min/1.73 m²) |
|---|---|---|---|
| Mean | 32.1 | 32.5 | + 0.41 |
| Median | 29.8 | 29.8 | 0 |
| Min, Max | 6.1, 69.5 | 6.1, 63.5 | - 18.3, + 24 |

Based on these results it can be concluded that long-term subcutaneous administration of C1-INH according to the dosing regimen disclosed herein is safe and an increase of the mean eGFR of +0.41 mL/min/1.73 m² was observed within the treated patient population. It is known that eGFR generally decreases in rAMR and based on the medical literature. Patients with AMR that do not improve upon conventional treatment, e.g. with IVIg/Plasmapheresis, typically lose eGFR - the lack of effective treatments in these situations and the consequences have been described in literature. The rate reported in literature is about - 4 to - 8 mL/min/1.73 m²/year which corresponds to a loss of 1 to 2 mL/min/1.73 m² over 12 weeks (An et al., J. Immunol. Res. 2014; 2014(828732), 1-7; Sablik et al., BMC Nephrol. 2019; 20(1): 218; Eskandary et al., Am. Soc. Nephrol. 2018; 29(2): 591-605; Böhmig et al., Transpl. Int. 2019; 32(8): 775-788; Larpparisuth et al., Transplant Proc. 2019; 51(10): 3293-3296). eGFR is a known surrogate marker for graft loss and success in treatment of AMR (Clayton et al., J. Am. Soc. Nephrol. 2016; 27(11): 3440-3446.; Böhmig et al., Transpl Int. 2019; 32(8): 775-788). A lesser degree of decline in eGFR is associated with a better graft function and longer graft life.

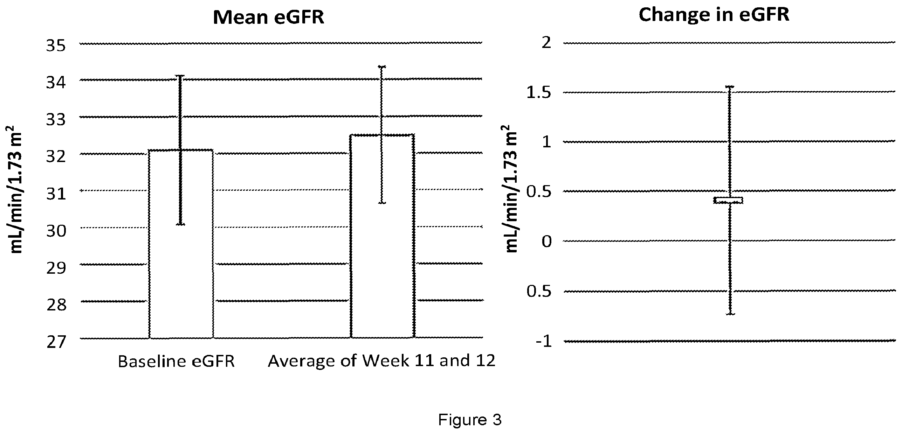

With this study it was found that eGFR instead of decreasing as previously reported, on average it increased by a mean of 0.41 mL/min/1.73 m² in the patient group. This increase is shown in Figure 3. In conclusion, the patient group having received the treatment described herein exhibit improved kidney function compared to historical controls.

### EXAMPLE 2: A Double-blind, Randomized-withdrawal, Placebo-controlled Study to Evaluate the Efficacy and Safety of Human Plasma-derived C1 inhibitor as add-on to Standard of Care for the Treatment of Refractory Antibody Mediated Rejection in Adult Renal Transplant Recipients.

### Investigational Product and Eligibility Criteria

The investigational product and eligibility criteria for patient selection are identical to EXAMPLE 1. Placebo will be manufactured in accordance with ICH Good Manufacturing Practice guidelines and local regulatory requirements. It will be provided as a lyophilized powder for reconstitution. Before use, each vial of Placebo is reconstituted with 3 mL water for injection. The actual dose of placebo is rounded to the nearest lower practical volume of 1 mL. A dose of 60 IU/kg C1-INH is equivalent to a volume of 0.12 mL/kg and placebo is also injected subcutaneously at a dose of 0.12 mL/kg according to the same schedule as C1-INH. Vials of C1-INH and placebo are packaged identically and individual packages may be identified only by the kit number.

### Study Design and Interventions

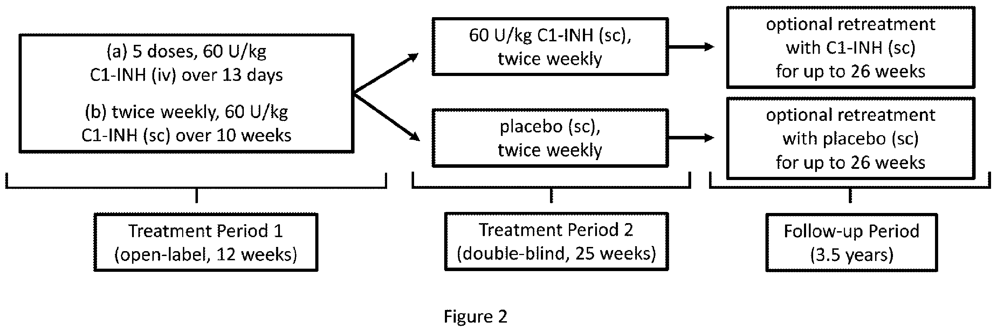

This study is divided into phases: an open labeled Treatment Period 1, a closed labeled Treatment Period 2 and a Follow-up Period with optional retreatment. Figure 2 shows these phases.

Treatment Period 1 will be conducted in accordance with the treatment of EXAMPLE 1 (same dosing regimen and other interventions). However, different from EXAMPLE 1 instead of 47 patients, approximately 120 subjects are planned to participate in this open-label Treatment Period 1. It should be noted that this EXAMPLE 2 is a continuation of the study of EXAMPLE 1, i.e. the 120 patients of EXAMPLE 2 may comprise the patients of EXAMPLE 1 (EXAMPLE 1 would correspond to Treatment Period 1). Only patients that respond to treatment of open labeled Treatment Period 1 (Responders) will participate in closed labeled, randomized Treatment Period 2. Responders must have an eGFR value that is equal or above 20 mL/min/1.73 m², AND equal or above 90% of baseline eGFR value at the end of Treatment Period 1. Non-responders will not participate in Treatment Period 2 in order to exclude patients that do not respond for whatever reason to the described treatment with C1-INH of Treatment Period 1. For those that stay in the study, the effect of even longer administration of C1-INH will be studied.

During Treatment Period 2 eligible subjects will be randomized 1 : 1 to receive treatment with blinded investigational product (C1-INH (60 IU/kg) or placebo) subcutaneously about twice weekly for 26 weeks. Treatment Period 1 (12 weeks) and Treatment Period 2 (26 weeks) amount to a combined treatment duration of 38 weeks. All subjects will receive IVIg 2 grams/kg once every 4 weeks throughout Treatment Periods 1 and optional through Treatment Period 2. Plasmapheresis will be implemented, as needed.

At the end of Treatment Period 2 at Week 38 subjects will enter a ~3.5-year post-treatment Follow-up Period. Subjects will receive treatment consistent with their local standard of care during the Follow-up Period, and their allograft status and survival will be monitored. Subjects may undergo retreatment during the post-treatment Follow-up Period with the blinded investigational product to which they were randomized in Treatment Period 2. Retreatment may occur following a diagnosis of recurrent by AMR by biopsy. Retreatment may start at any time during the post-treatment Follow-up Period, and will last for 26 weeks or less. The different treatment periods of EXAMPLE 2 are shown in Table 2.

**Table 2: Dosing regimen for investigational product**

| | |
|---|---|
| Treatment Period 1 (a) | 60 IU/kg C1-INH intravenously on Days 1, 4, 7, 10, 13 |
| Day 1 to Day 13 | |
| Treatment Period 1 (b) | 60 IU/kg C1-INH subcutaneously about twice weekly |
| Day 14 to Week 12 | |
| Treatment Period 2 | 60 IU/kg C1-INH about twice weekly subcutaneously |
| Week 13 to Week 38 | OR |
| | 0.12 mL/kg Placebo about twice weekly subcutaneously |
| Optional Retreatment Period | 60 IU/kg C1-INH about twice weekly subcutaneously |
| | OR |
| Retreatment Day 1 to Retreatment Week 26 | 0.12 mL/kg Placebo about twice weekly subcutaneously |

The duration of the study for an individual subject is expected to be approximately 210 weeks (~ 4 years). This includes the Treatment Period 1 and 2 as well as the post-treatment Follow-up Period of 3.5 years. The primary objective of the study is to further confirm the long-term efficacy of C1-INH in the treatment of refractory AMR in renal allograft recipients. A secondary objective is to further study the pharmacokinetics of C1-INH during the treatment of refractory AMR in renal allograft recipients.

In some embodiments, kidney function reflected by the stabilization of eGFR, and incidence of subject survival and allograft survival may be better for the group that receives C1-INH. In some patients, eventual graft loss may happen less often or take longer to occur in the C1-INH group than in the placebo group. The Follow-up Period will be used to assess graft loss in the subjects.

## Claims

1. A C1-INH for use in a method of treating antibody-mediated rejection in a transplant recipient comprising administering C1-INH to the recipient according to a schedule comprising the following steps:
(a) intravenously administering one or more iv-doses of C1-INH, then
(b) subcutaneously administering C1-INH over at least 10 weeks, wherein each week at least one sc-dose is administered.

2. The C1-INH of claim 1, wherein the C1-INH is human plasma-derived C1-INH.

3. The C1-INH of claim 1 or 2, wherein each iv-dose and/or each sc-dose contains 40 to 120 IU/kg C1-INH.

4. The C1-INH of any one of claims 1-3, wherein, in step (b), at least 20 sc-doses of C1-INH are administered.

5. The C1-INH of any one of claims 1-4, wherein 3 to 5 iv-doses of C1-INH are administered over 7 to 13 days.

6. The C1-INH of any one of claims 1-5, wherein the iv-doses are administered every second, third or fourth day and/or wherein the sc-doses of C1-INH are administered about twice or about three times weekly.

7. The C1-INH of any one of claims 1-6, wherein a total amount of 7,000 to 36,000 IU C1-INH is administered intravenously in step (a) and/or wherein an amount of 4,000 to 15,000 IU C1-INH is administered weekly in step (b).

8. The C1-INH of any one of claims 1-7, wherein the sc-doses are administered over a period of time that is at least 5 times as long as a period of time for administration of the iv-doses.

9. The C1-INH of any one of claims 1-8 wherein at least twice as much C1-INH is administered subcutaneously as is administered intravenously.

10. The C1-INH of any one of claims 1-9, wherein more than 50,000 IU C1-INH is administered in total, in particular wherein more than 100,000 IU C1-INH is administered in total.

11. The C1-INH of any one of claims 1-10, wherein the formulations of the C1-INH for the iv-doses and the sc-doses are identical and/or wherein each iv-dose comprises the same amount of C1-INH as each sc-dose.

12. The C1-INH of any one of claims 1-11, wherein the C1-INH is administered intravenously and/or subcutaneously at a concentration of 200 to 800 IU/mL.

13. The C1-INH of any one of claims 1-12, wherein the recipient is an allograft recipient, in particular a kidney transplant recipient.

14. The C1-INH of any one of claims 1-13, wherein the method is initiated after transplantation, in particular more than three months after transplantation.

15. The C1-INH of any one of claims 1-14, wherein the antibody-mediated rejection is refractory antibody-mediated rejection.

16. The C1-INH of any one of claims 1-15, wherein IVIg is administered in addition to administration of the C1-INH, in particular wherein the IVIg is administered as an infusion every 3 to 5 weeks, wherein each infusion comprises 0.1 to 2 g/kg IVIg.

17. A C1-INH for use in a method of treating antibody-mediated rejection in a transplant recipient comprising subcutaneously administering C1-INH over at least 10 weeks, wherein each week at least one sc-dose is administered, wherein at least 20 sc-doses of C1-INH are administered.

## Patentansprüche

1. C1-INH zur Verwendung in einem Verfahren zur Behandlung von antikörpervermittelter Abstoßung in einem Transplantatempfänger, umfassend Verabreichen von C1-INH an den Empfänger gemäß einem Zeitplan, der die folgenden Schritte umfasst:
(a) intravenöse Verabreichung einer oder mehrerer iv-Dosen von C1-INH, dann
(b) subkutane Verabreichung von C1-INH über mindestens 10 Wochen, wobei jede Woche mindestens eine sc-Dosis verabreicht wird.

2. C1-INH nach Anspruch 1, wobei es sich bei dem C1-INH um von menschlichem Plasma abgeleitetes C1-INH handelt.

3. C1-INH nach Anspruch 1 oder 2, wobei jede iv-Dosis und/oder jede sc-Dosis 40 bis 120 IU/kg C1-INH enthält.

4. C1-INH nach einem der Ansprüche 1-3, wobei in Schritt (b) mindestens 20 sc-Dosen C1-INH verabreicht werden.

5. C1-INH nach einem der Ansprüche 1-4, wobei über 7 bis 13 Tage 3 bis 5 iv-Dosen C1-INH verabreicht werden.

6. C1-INH nach einem der Ansprüche 1-5, wobei die iv-Dosen jeden zweiten, dritten oder vierten Tag verabreicht werden, und/oder wobei die sc-Dosen von C1-INH etwa zweimal oder etwa dreimal wöchentlich verabreicht werden.

7. C1-INH nach einem der Ansprüche 1-6, wobei in Schritt (a) eine Gesamtmenge von 7.000 bis 36.000 IU C1-INH intravenös verabreicht wird, und/oder wobei in Schritt (b) wöchentlich eine Menge von 4.000 bis 15.000 IU C1-INH verabreicht wird.

8. C1-INH nach einem der Ansprüche 1-7, wobei die sc-Dosen über einen Zeitraum verabreicht werden, der mindestens 5 mal so lang wie ein Zeitraum für die Verabreichung der iv-Dosen ist.

9. C1-INH nach einem der Ansprüche 1-8, wobei mindestens doppelt so viel C1-INH subkutan verabreicht wird wie intravenös verabreicht wird.

10. C1-INH nach einem der Ansprüche 1-9, wobei insgesamt mehr als 50.000 IU C1-INH verabreicht werden, wobei insbesondere insgesamt mehr als 100.000 IU C1-INH verabreicht werden.

11. C1-INH nach einem der Ansprüche 1-10, wobei die Formulierungen des C1-INH für die iv-Dosen und die sc-Dosen identisch sind, und/oder wobei jede iv-Dosis die gleiche Menge an C1-INH wie jede sc-Dosis umfasst.

12. C1-INH nach einem der Ansprüche 1-11, wobei das C1-INH intravenös und/oder subkutan in einer Konzentration von 200 bis 800 IU/ml verabreicht wird.

13. C1-INH nach einem der Ansprüche 1-12, wobei der Empfänger ein Allotransplantatempfänger, insbesondere ein Nierentransplantatempfänger, ist.

14. C1-INH nach einem der Ansprüche 1-13, wobei das Verfahren nach der Transplantation, insbesondere mehr als drei Monate nach der Transplantation, initiiert wird.

15. C1-INH nach einem der Ansprüche 1-14, wobei die antikörpervermittelte Abstoßung refraktäre antikörpervermittelte Abstoßung ist.

16. C1-INH nach einem der Ansprüche 1-15, wobei IVIg zusätzlich zur Verabreichung des C1-INH verabreicht wird, wobei insbesondere das IVIg als Infusion alle 3 bis 5 Wochen verabreicht wird, wobei jede Infusion 0,1 bis 2 g/kg IVIg umfasst.

17. C1-INH zur Verwendung in einem Verfahren zur Behandlung von antikörpervermittelter Abstoßung in einem Transplantatempfänger, umfassend subkutane Verabreichung von C1-INH über mindestens 10 Wochen, wobei jede Woche mindestens eine sc-Dosis verabreicht wird, wobei mindestens 20 sc-Dosen C1-INH verabreicht werden.

## Revendications

1. C1-INH destiné à être utilisé dans un procédé de traitement d'un rejet médié par des anticorps chez un receveur de greffe, comprenant l'administration de C1-INH au receveur selon un plan comprenant les étapes suivantes:
(a) administration par voie intraveineuse d'une ou plusieurs doses iv de C1-INH, puis
(b) administration par voie sous-cutanée de C1-INH pendant au moins 10 semaines, chaque semaine au moins une dose sc étant administrée.

2. C1-INH selon la revendication 1, le C1-INH étant du C1-INH dérivé du plasma humain.

3. C1-INH selon la revendication 1 ou 2, chaque dose iv et/ou chaque dose sc contenant 40 à 120 UI/kg de C1-INH.

4. C1-INH selon l'une quelconque des revendications 1 à 3, dans l'étape (b), au moins 20 doses sc de C1-INH étant administrées.

5. C1-INH selon l'une quelconque des revendications 1 à 4, 3 à 5 doses iv de C1-INH étant administrées en 7 à 13 jours.

6. C1-INH selon l'une quelconque des revendications 1 à 5, les doses iv étant administrées tous les deuxième, troisième ou quatrième jours et/ou les doses sc de C1-INH étant administrées environ deux fois ou environ trois fois par semaine.

7. C1-INH selon l'une quelconque des revendications 1 à 6, une quantité totale de 7 000 à 36 000 UI de C1-INH étant administrée par voie intraveineuse dans l'étape (a) et/ou une quantité de 4 000 à 15 000 UI de C1-INH étant administrée chaque semaine dans l'étape (b).

8. C1-INH selon l'une quelconque des revendications 1 à 7, les doses sc étant administrées sur une période de temps qui est au moins 5 fois plus longue qu'une période de temps pour l'administration des doses iv.

9. C1-INH selon l'une quelconque des revendications 1 à 8, au moins deux fois plus de C1-INH étant administré par voie sous-cutanée que par voie intraveineuse.

10. C1-INH selon l'une quelconque des revendications 1 à 9, plus de 50 000 UI de C1-INH étant administré au total, en particulier plus de 100 000 UI de C1-INH étant administré au total.

11. C1-INH selon l'une quelconque des revendications 1 à 10, les formulations du C1-INH pour les doses iv et les doses sc étant identiques et/ou chaque dose iv comprenant la même quantité de C1-INH que chaque dose sc.

12. C1-INH selon l'une quelconque des revendications 1 à 11, le C1-INH étant administré par voie intraveineuse et/ou sous-cutanée à une concentration de 200 à 800 UI/ml.

13. C1-INH selon l'une quelconque des revendications 1 à 12, le receveur étant un receveur d'allogreffe, en particulier un receveur de greffe rénale.

14. C1-INH selon l'une quelconque des revendications 1 à 13, le procédé étant initié après transplantation, en particulier plus de trois mois après transplantation.

15. C1-INH selon l'une quelconque des revendications 1 à 14, le rejet médié par les anticorps étant un rejet médié par les anticorps réfractaires.

16. C1-INH selon l'une quelconque des revendications 1 à 15, IVIg étant administré en plus de l'administration de C1-INH, en particulier l'IVIg étant administré sous forme d'une perfusion toutes les 3 à 5 semaines, chaque perfusion comprenant 0,1 à 2 g/kg de IVIg.

17. C1-INH destiné à être utilisé dans un procédé de traitement d'un rejet médié par des anticorps chez un receveur de greffe, comprenant l'administration sous-cutanée de C1-INH pendant au moins 10 semaines, chaque semaine au moins une dose sc étant administrée, au moins 20 doses sc de C1-INH étant administrées.
